# EUROPEAN PATENT APPLICATION

(11) **EP 1 421 963 A1**
(43) Date of publication of application: **26.05.2004**
(21) Application number: 03022070.1
(22) Date of filing: 02.10.2003
(51) Int. Cl.: A61M 5/32

(54) **Safety syringe with retractable needle having a soft piston**

(30) Priority: 22.11.2002 US 301577
(71) Applicant: Life-Shields Products, Inc., Wugu Shiang, Taipei (TW)
(72) Inventor: Wu, Peng-Chieh, San Chung City Taipei (TW)
(74) Representative: Meyer, Ludgerus A.

(57) **Abstract**

The retractable safety syringe for drawing back a soft piston therein is comprised of a needle set, a cylindrical barrel and a plunger. A retractable mechanism is provided for pulling the needle set into it. The plunger is provided on the front end thereof with a soft piston, and with an activating piece to activate the retractable mechanism or with a connecting piece connectable to the retractable mechanism. When in assembling, the soft piston of a plunger is pushed to the top point of a space provided for injection, before deformation of the piston, an activating piece or a connecting piece on the plunger does not activate or connect with the retractable mechanism, and when the plunger is pushed by a force larger than a preset amount, the piston is compressed to deform and to render the activating piece to activate or connect with the retractable mechanism to pull the needle set into the cylindrical barrel automatically or manually.

## Description

### BACKGROUND OF THE INVENTION

### 1. Field of the invention

The present invention is related to a retractable safety syringe with a soft piston; and especially to such a syringe in which the piston is made of elastic material in order that when in drawing in and injecting medicine, the front end face of the piston can be pushed to the top end of the cylindrical barrel of the syringe to allow accurate taking out of the medicine following the indicating scale marks and pressing the medicine out stably; and after injecting, the piston can be deformed by pressing, so that a connecting piece or an activating piece provided on a plunger can connect or activate a retractable mechanism provided on a needle set, and the needle set can be retracted into the cylindrical barrel. The syringe suits various cylindrical barrels of syringes or similar structures.

### 2. Description of the Prior Art

Since the medical science was widely developed, medicine injection with syringes has been the fastest and most effective way of treatment, and it is widely adopted in the medical field. However, syringes are generally discarded after use, it becomes the main point of designing for the goal of preventing accidental stabbing and infecting medical workers in dealing with waste syringes, and the goal of reducing the volumes of discarded syringes. Thereby, various retractable safety syringes have been developed.

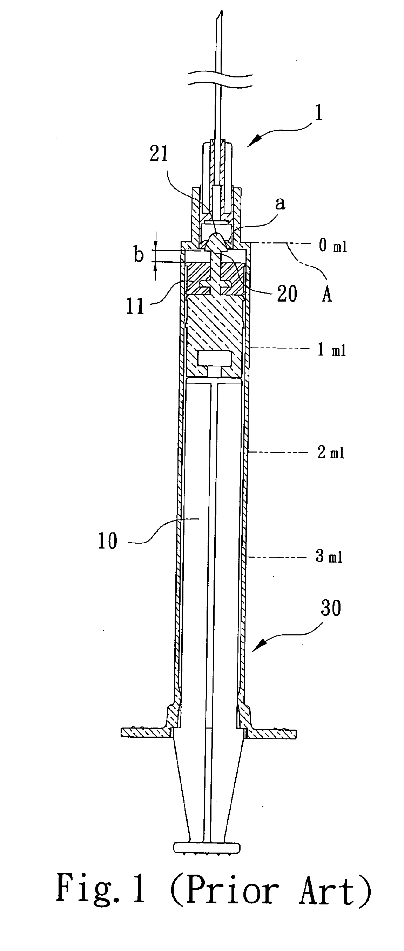

Referring to Fig. 1 showing the structure of a conventional manual retractable safety syringe, wherein a plunger 10 can be pulled or pushed in a cylindrical barrel 30; the plunger 10 is provided on the front end thereof with a piston 11 and a hook portion 21, the hook portion 21 is protruded from the front end of piston 11 to form a neck portion 20 with a length "b" therebetween. A connecting portion "a" is provided on the bottom of a needle set 1, when the plunger 10 is pushed forwards to the top point, the hook portion 21 can pierce into the connecting portion "a" to form a hooking state therewith, thereby the needle set 1 can be pulled back into the cylindrical barrel 30.

The connecting portion "a" of the needle set 1 shown in Fig. 1 is located at the center of the bottom of the needle set 1; the hook portion 21 is also located at the center of the plunger 10 to form a connecting piece. However, there are other retractable mechanisms with different pulling back modes for conventional manual retractable safety syringes. For example, the connecting portion of the needle set can be provided on the exterior surface of the base of the needle set, and the connecting piece of the plunger can be provided on the exterior surface of the plunger to be able to mutually hook or engage.

Whichever style a manual retractable safety syringe is, generally, to avoid the connecting portion "a" of the plunger 10 to be pushed inadvertently forwards to the top point before discarding the cylindrical barrel 30 of the syringe, a measure is adopted to render the plunger 10 to be moved only when a sufficient force is exerted thereon to make connection of the connecting piece with the connecting portion "a" at the moment when the front end of the plunger 10 contacts the base of the needle set 1.

There are also automatic retractable safety syringes taking advantage of the restoring force of springs, such automatic retractable safety syringes shall have their activating pieces provided on the plungers also moved by forces to overcome resistances of predetermined values before the spring retractable mechanisms are activated.

Medical workers generally pull backwards the push rods on the rear ends of plungers of syringes to draw air into the cylindrical barrels, and then pierce needles into the medicine bottles and push forwards the plungers. The air drawn into the cylindrical barrels is injected into the medicine bottles, and the plungers are pulled back following the indicating marks to draw required amount of medicine into the cylindrical barrels. And when in injection for patients, to avoid remaining of the medicine in the cylindrical barrels, the medical workers generally will push the push rods forwards to the top points. Hence, whichever of the manual or automatic retractable safety syringes they are, during the process of injection, forces must be exerted to overcome the resistances in connecting and activating of the retractable mechanisms finally.

The above stated conventional retractable safety syringes always have following defects:
1. As shown in Fig. 1, there is a distance "b" (i.e., the length of the neck portion 20) left between the piston 11 and the hook portion 21, thereby, when in taking injection liquid before injection, the amount of the injection medicine taken will be reduced. That is to say, the medical workers must increase the amount taken when in drawing the medicine for injection in order to avoid reducing of the treatment efficiency due to insufficiency of medicine; and if the medicine for injection is insufficient due to neglect of the medical workers, the treatment efficiency will also be lowered to influence development in treating patients.
2. When in injecting medicine for a patient with a syringe by a medical worker, by the fact that the front end of the piston 11 is unable to reach the position where the top mark indication line "A" showing the amount of 0 ml is, while there is still injection liquid in the space "b" between the piston 11 and the hook portion 21, the medical worker shall exert a force to push the plunger 10 to the top point, such force added abruptly during injecting may create a pain feeling of a patient.

In view of these, the inventor of the present invention studied and developed a retractable safety syringe for drawing back a soft piston that can accurately draw in the required medicine and can stably push the plunger to the top point; and when the needle is drawn out of the body of a patient after injection, a force can be exerted to push forwards and draw back the plunger.

### SUMMARY OF THE INVENTION

The primary object of the present invention is to provide a retractable safety syringe for drawing back a soft piston, with the soft piston and such a designing having the distance between the front end of the piston and the connecting piece or the activating piece of the plunger reduced, the amount of medicine taken can be accurate, so that when in injection, no abrupt adding of force is required to push the plunger to the top point, and a patient can be prevented from getting a pain feeling.

The secondary object of the present invention is to provide a retractable safety syringe that can reduce the residual medicine amount in the cylindrical barrel of the syringe.

To get the above objects, the present invention is comprised of a needle set, a cylindrical barrel and a plunger. An automatic retractable mechanism or a manual retractable mechanism is provided thereon the needle set for pulling the needle set into the cylindrical barrel. The plunger is provided on the front end thereof with a soft piston, and with an activating piece to activate the automatic retractable mechanism for the needle set or with a connecting piece connectable to the manual retractable mechanism for the needle set. The plunger is inserted into the cylindrical barrel from the rear end of the latter, the cylindrical barrel is a hollow barrel, and the upper part thereof is formed a needle set socket to allow insertion connecting of the needle set, while the lower part thereof is a space provided for injection. The piston is made of elastic material; the connecting piece or the activating piece on the front end of the plunger has its bottom surface exactly protruded out of the front end face of the piston. When in the state of injection, the upper surface of the piston is contacted with the bottom surface of the connecting piece or the activating piece. Hence when in injection, the plunger is stably pushed to the top point to completely eject all the medicine; and when in discarding, the piston is pushed to move the connecting piece or the activating piece forwardly to be released from contact with the upper surface of the piston and to push it into the retractable mechanism to activate the automatic retractable mechanism or to connect it with the connecting portion of the manual retractable mechanism, to thereby pull the needle set down into the cylindrical barrel.

The present invention will be apparent in its features and structure after reading the detailed description of the preferred embodiment thereof in reference to the accompanying drawings.

### BRIEF DESCRIPTION OF THE DRAWINGS

Fig. 1 is a sectional view showing the structure of a conventional syringe;
Fig. 2 is an analytic perspective view showing the elements of the embodiment of the present invention;
Fig. 3 is a sectional view of the embodiment of the present invention;
Fig. 4 is a sectional view showing drawing in the liquid for injection by the embodiment of the present invention;
Fig. 5a is a sectional view showing a hook portion engages an engaging ratchet in the embodiment of the present invention;
Fig. 5b is an enlarged sectional view showing the hook portion engages the engaging ratchet in the embodiment of the present invention;

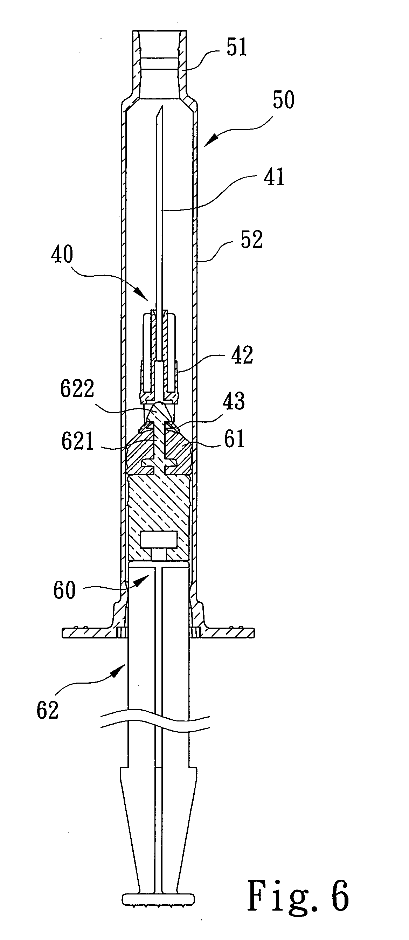

Fig. 6 is a sectional view of the embodiment of the present invention showing drawing back of a needle set;
Fig. 7a is a coordinate plane showing the force in injecting medicine from N ml to 0 ml by the retractable safety syringe of the present invention;
Fig. 7b is a coordinate plane showing the force in injecting medicine from N ml to 0 ml by the retractable safety syringe of the conventional syringe;
Fig. 8 is a schematic sectional view showing another embodiment having an extended protrusion on the top end of the hook portion of the present invention;
Fig. 9 is an enlarged sectional view showing the hook portion of Fig. 8 is engaged with an engaging ratchet of the present invention.
Fig. 10 is a partly sectional view showing a further example of the prevent invention applying to a needle set comprising a needle, needle seat and retractable adapter.

### DETAILED DESCRIPTION OF THE PREFERRED EMBODIMENT

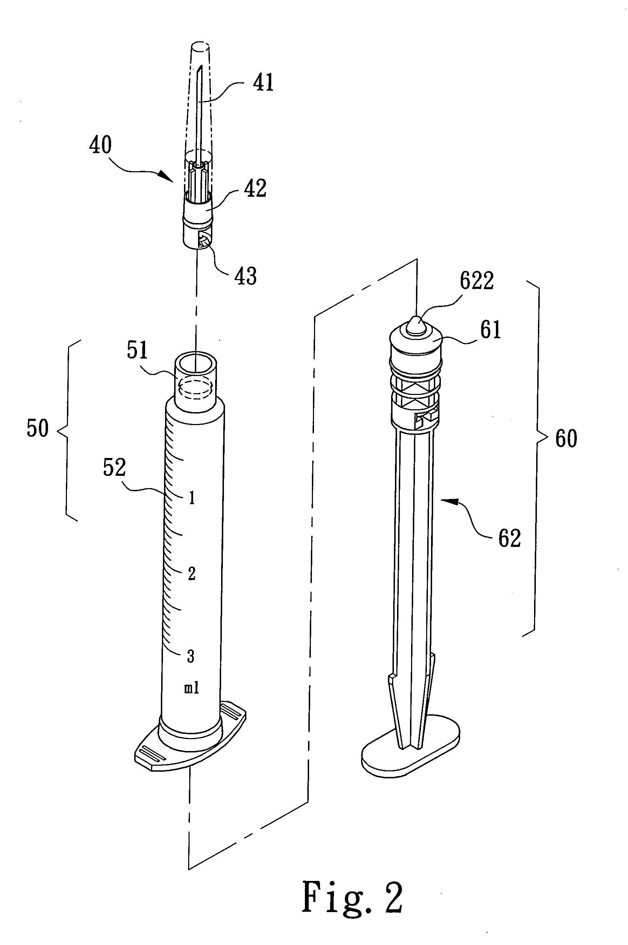

Referring to Fig. 2 showing an embodiment of a manual retractable safety syringe of the present invention having a soft piston, the syringe is comprised of a needle set 40, a cylindrical barrel 50 and a plunger 60.

Wherein, the needle set 40 is comprised of a needle 41 and a needle seat 42 for fixing therein the needle 41; the needle seat 42 is in the form of a hollow pipe and is provided therein with an engaging ratchet 43. A hollow shaft of the needle 41 and the central portion of the needle seat 42 are both hollow and are communicated with the central portion of the engaging ratchet 43, such hollow portions form a passing space for medicine.

The cylindrical barrel 50 is a hollow cylinder, a space 52 for injection is provided on the rear part of the cylindrical barrel 50, and scale marks (taking ml as the unit) indicating the amounts of medicine are printed on the external surface of the cylindrical barrel 50. The cylindrical barrel 50 is formed on the front end thereof a needle set socket 51 to receive the needle seat 42 of needle set 40.

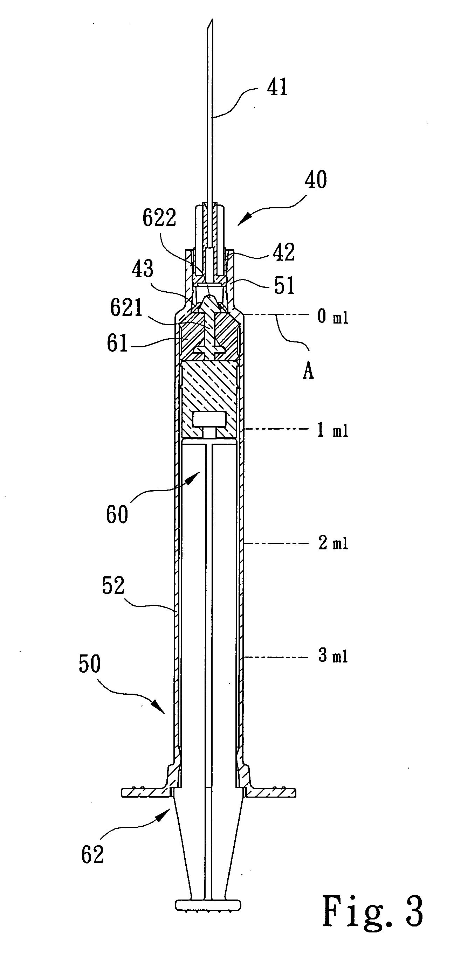

The plunger 60 is comprised of a piston 61 and a pusher rod 62; the piston 61 is made of elastic material. The pusher rod 62 is provided on the front end thereof with a piston shaft 621 (referring to Fig. 3); the piston shaft 621 is provided on the top end thereof with a hook portion 622, the piston 61 is slipped over the piston shaft 621.

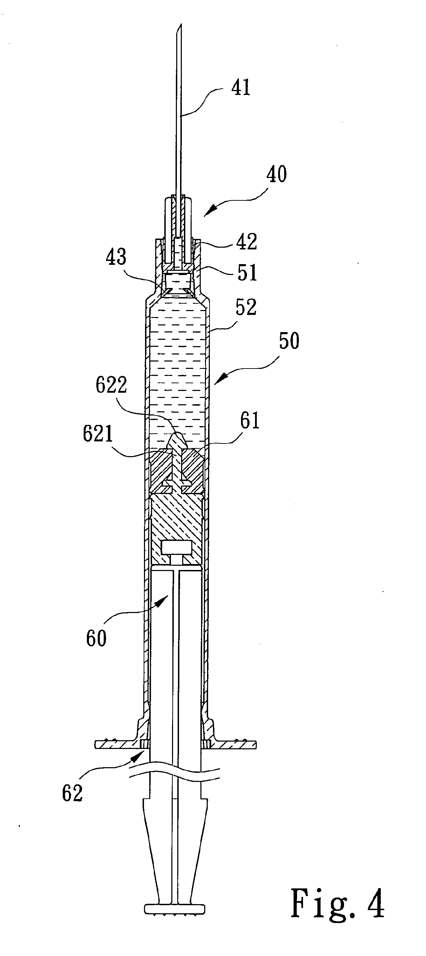

As shown in Figs. 2, 3, the piston 61 in this embodiment is made of elastic material, the piston shaft 621 on the front end of the pusher rod 62 has a length equal to the thickness of the piston 61; so that when the piston 61 is extended over the piston shaft 621, only the hook portion 622 of the piston shaft 621 protrudes out of the piston 61 to render the bottom end of the hook portion 622 to contact the piston 61. Referring to Figs. 3, 4, when the pusher rod 62 is pulled back to draw in medicine for injection, the front end face of the piston 61 is at the position where the top scale mark "A" (indicating 0 ml) is, hence medicine can be drawn in with accurate amount.

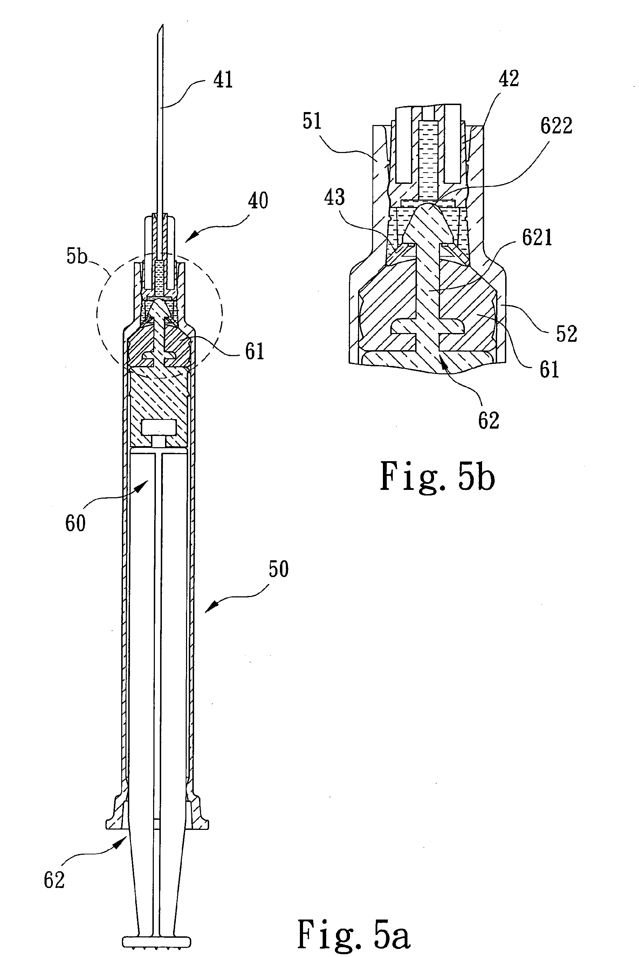

When in injecting medicine, by virtue that the front end face of the piston 61 contacts with the bottom end of the hook portion 622, the plunger 60 can be stably pushed to a top point to completely eject all the medicine; and when in discarding a syringe, as shown in Figs. 5a and 5b, the piston 61 is pushed and compressed to deform, thereby the hook portion 622 of the piston shaft 621 is released from contact with the upper surface of the piston 61 to in turn push forwards the engaging ratchet 43 of the needle seat 42 and to engage with the engaging ratchet 43. And the needle set 40 can be pulled down into the cylindrical barrel 50.

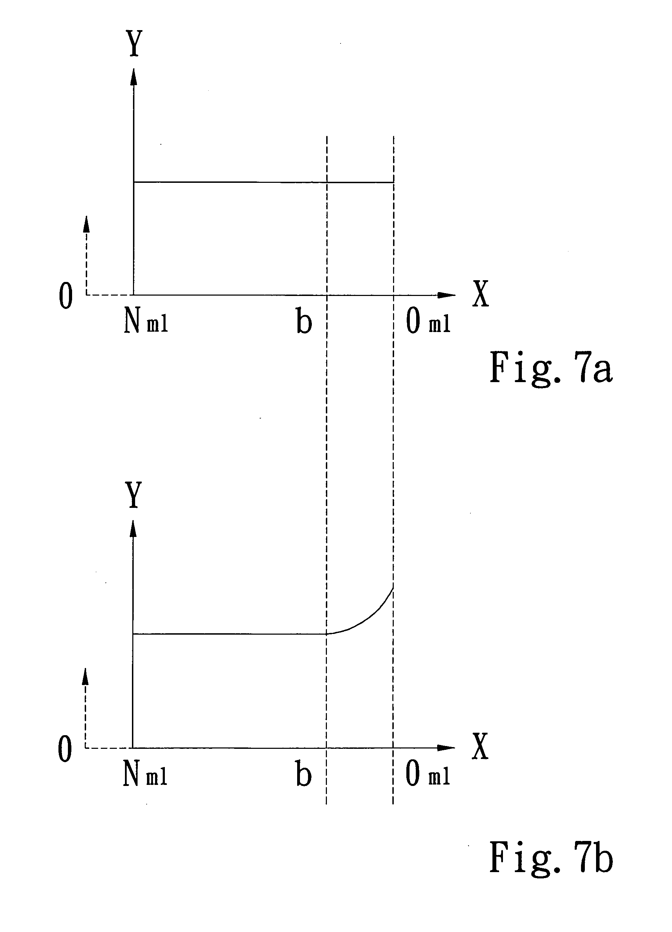

And referring to Figs. 7a and 7b that respectively provide coordinate planes showing the force in injecting medicine by the retractable safety syringe of the present invention and of the conventional syringe, wherein the Y axis indicates the force that a medical worker exerts, while the X axis indicates the residual amount of medicine, the starting point represents that the amount of medicine in the cylindrical barrel is N ml, the amount is gradually reduced to 0 ml.

The retractable safety syringe of the present invention and the conventional retractable safety syringe are both poured in with N ml medicine. When in injection, before the residual amount of medicine is b ml, the retractable safety syringes of the present invention and the conventional one are exerted with forces of the same amount; and when injection is proceeded to having the residual amount of medicine of b ml, as for the conventional retractable safety syringe, the hook portion of the plunger thereof is pushed upwardly to contact the engaging ratchet on the bottom of the needle seat and is hindered from forwarding, the medical worker must put a force to eject the remaining medicine, increasing of force makes pain of a patient; while as for the present invention, force is stably exerted, no additional force is necessary to push the piston to the top point but injecting of medicine can be completed.

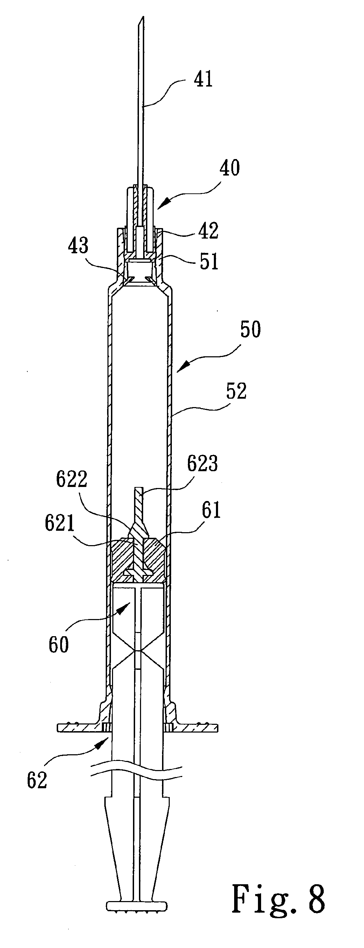

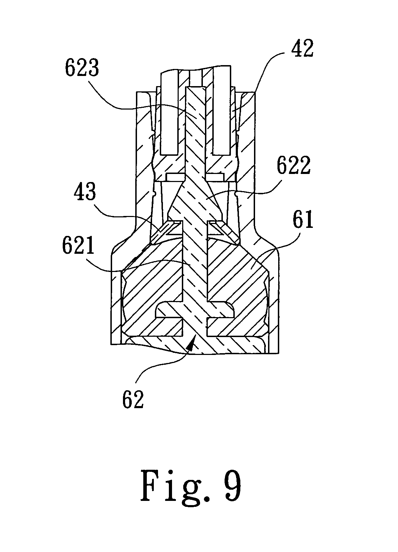

Referring to Figs. 8, 9, in the above stated embodiment of the present invention, an extended protrusion 623 can also be formed on the front end of the hook portion 622, so that when the hook portion 622 of the piston shaft 621 is pierced into the engaging ratchet 43 of the needle seat 42 to form an engagement state, the extended protrusion 623 formed on the front end of the hook portion 622 can be extended deeply into the needle seat 42 to occupy the space therein, thereby the residual amount of injection liquid can be further lowered.

In the drawings of the present invention, although the needle set socket 51 of the cylindrical barrel 50 is in the state of having a reduced diameter, if the needle set 40 is of the structure of which the needle 41 is changeable, the needle set socket 51 of the cylindrical barrel 50 can also have its bottom enlarged to be in the form of a straight barrel to allow the needle set socket 51 and the cylindrical barrel 50 to have the same inner diameter, but the needle set 40 can be in the same way inserted for connection.

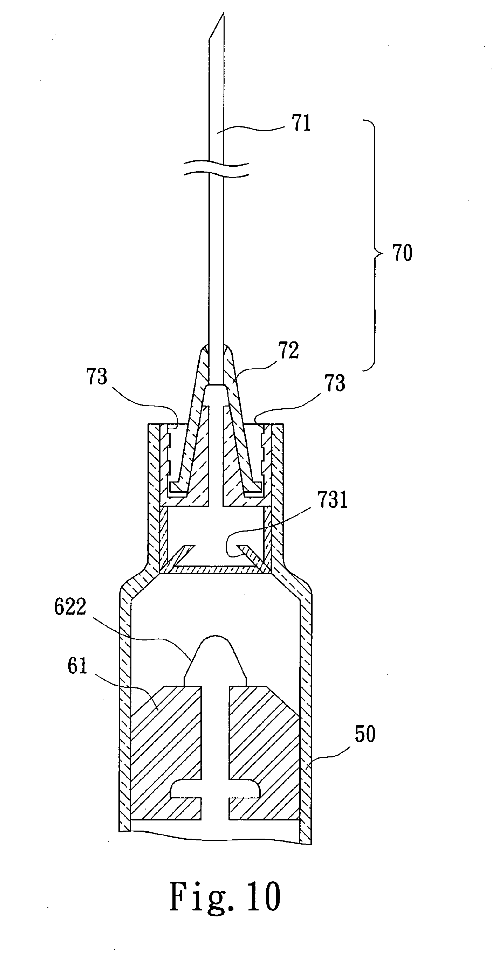

Fig. 10 is shown a further application of the present invention, wherein the needle set 70 is comprised of a needle 71, a needle seat 72, and a retractable adapter 73 having a retractable mechanism, such as an engaging ratchet 731.

And in the above stated drawings and specification, the engaging ratchet 43 on the base of the needle set 40 forms a connecting portion of a manual retractable mechanism, in which the shape of the arrangement the engaging ratchet 43 and the arrangement that the engaging ratchet 43 is located at the center of the base of the needle set 40 only form an embodiment of such a manual retractable safety syringe. In fact, the structure of the soft piston and the connecting piece of the plunger of the present invention can be applied to any manual retractable safety syringe having a connecting piece of the plunger and a connecting portion of the needle set of any of various shapes and at any of various locations. The structure of the soft piston and the connecting piece of the plunger of the present invention can also be applied to an automatic retractable safety syringe; in which the connecting piece of the plunger forms the activating piece to activate the retractable mechanism provided on the needle set.

In conclusion, the present invention surely provides an improved structure for the connecting piece or the activating piece for a piston and a retractable mechanism for use on a retractable syringe; the present invention thereby is industrially practical.

It will be apparent to those skilled in this art that various modifications or changes can be made to the elements of the present invention without departing from the spirit and scope of this invention; all such modifications and changes shall fall within the scope of the appended claims and are intended to form part of this invention.

## Claims

1. A retractable safety syringe for drawing back a soft piston, said syringe comprises:
a needle set, at least including a needle and a needle seat, being provided thereunder with a retractable mechanism for pulling said needle set into it;
a cylindrical barrel, the upper part thereof being formed a needle set socket, the lower part thereof being a space provided for injection, said needle set being connected onto said needle set socket;
a plunger being inserted into said space provided for injection in said cylindrical barrel from the rear end of said cylindrical barrel, and being provided on the front end thereof with a piston and with a connecting piece adapted to connecting to said retractable mechanism, said piston being a soft piston to be pushed and compressed to deform;
when in assembling, the top point of said space provided for injection before deformation of said piston prevents said connecting piece on said plunger from connecting with said retractable mechanism, and when said plunger is pushed by a force larger than a preset amount, said piston is compressed to deform and to displace said connecting piece to connect with said retractable mechanism of said needle set.

2. The retractable safety syringe for drawing back a soft piston as claimed in claim 1, wherein said retractable mechanism of said needle set is provided on a base of said needle set with a connecting portion to make connection with said connecting piece of said plunger.

3. The retractable safety syringe for drawing back a soft piston as claimed in claim 2, wherein said connecting portion is an engaging ratchet formed at the central portion of said base, said connecting piece of said plunger is a hook portion protruded from the center of the front end of said piston, the bottom of said hook portion is contacted with the top surface of said piston, said hook portion is provided on the front end of a piston shaft, said piston shaft supports said piston and is provided on the front end of said plunger.

4. The retractable safety syringe for drawing back a soft piston as claimed in claim 3, wherein an extended protrusion is formed on the front end of said hook portion, when said hook portion is pierced into said engaging ratchet of said base of said needle set, said extended protrusion is extended into the space where injection liquid is.

5. The retractable safety syringe for drawing back a soft piston as claimed in claim 1, wherein the scale mark of injection liquid at said top point of said space provided for injection is zero.

6. A retractable safety syringe for drawing back a soft piston, said syringe comprises:
a needle set, at least including a needle and a needle seat, being provided therein with an automatic retractable mechanism for pulling said needle set into it;
a cylindrical barrel, the upper part thereof being formed a needle set socket, the lower part thereof being a space provided for injection, said needle set being connected onto said needle set socket;
a plunger being inserted into said space provided for injection in said cylindrical barrel from the rear end of said cylindrical barrel, and being provided on the front end thereof with a piston and with an activating piece to activate said automatic retractable mechanism, said piston being a soft piston to be pushed and compressed to deform;
when in assembling, the top point of said space provided for injection before deformation of said piston prevents said activating piece from activating said automatic retractable mechanism, and when said plunger is pushed by a force larger than a preset amount, said piston is compressed to deform and to move forwards said activating piece on said plunger to activate said automatic retractable mechanism to pull said needle set into said cylindrical barrel.
